# EUROPEAN PATENT APPLICATION

(11) **EP 4 091 674 A1**
(43) Date of publication of application: **23.11.2022**
(21) Application number: 22173610.1
(22) Date of filing: 16.05.2022
(51) Int. Cl.: A61Q 1/14, A61K 8/37, A61K 8/92

(54) **A NAIL POLISH REMOVER COMPOSITION AND A METHOD OF REMOVING POLISH FROM NAILS**

(30) Priority: 19.05.2021 IT 202100012887
(71) Applicant: Chromavis S.p.A., 26010 Offanengo (CR) (IT)
(72) Inventor: Fularska, Anna, Przasnysz (PL); Zielonka, Izabela, Kra nik (PL); Borzym, Justyna, P o sk (PL); Stosio, Milena, Pruszków (PL)
(74) Representative: Vanosi, Adelio Valeriano

(57) **Abstract**

The present invention relates to the technical fields of cosmetics and hygiene, in particular the removal of cosmetics, such as nail polishes. In particular, the present invention relates to a composition for removing nail polish, said composition being in the form of a cream or butter. The composition of the invention is also advantageously free of acetone, acetates, and alkylene carbonate. Moreover, this composition has a pleasant and natural odour, and is nonflammable.

The present invention also relates to a method of removing polish from nails by using said composition.

## Description

### FIELD OF THE INVENTION

The present invention relates to the technical field of cosmetics and hygiene, in particular the removal of cosmetics, such as nail polishes. In particular, the present invention relates to a composition for removing nail polish, said composition being in the form of a cream or butter. The present invention also relates to a method of removing polish from nails by using said composition.

### STATE OF THE ART

Nail polish removers are usually low-viscosity liquids which contain organic solvents as the main constituent.

They are used to remove polishes from fingernail and toenail by dissolving the nail polish to remove it from the surface. After the nail polish has been dissolved or softened, it is usually removed by rubbing action with a cotton pad.

Typically, the solvents contained in nail polish removers, such as acetone, ethyl acetate or butyl acetate, are volatile and flammable, with strong odour. Flammable formulations pose hazards during manufacture and use. These flammable formulations also present difficulties with regard to transport considerations as they are classified as dangerous goods. Furthermore, their strong odour may be considered unpleasant to user. Also, these solvents are not environmental -friendly. Last, these solvents have a dehydrating effect on the nails. This is disadvantageous, not only for the unpleasant feeling of itchy and dry nails after use, but also for the nail physiology, as dehydration causes brittle, splitting, and fragile nails.

Nail polish removers comprising carbonate as solvent, in particular propylene carbonate, are known. These are less odorous and do not leave the nails dry after use. However, their performances as nail polish removers have not been satisfactory so far.

In particular, all the nail polish removers in cream or cream-like form that are available on the market all contain polyethylene glycols (PEGs) or derivatives thereof as surfactants, solubilizing and stabilizing agents. The most common PEGs used are: PEG-40 Hydrogenated Castor Oil and PEG-60 hydrogenated castor oil.

Examples of nail polish remover in cream from the market are presented in Table 1 below.

**Table 1**

| | Prior art cream-like nail polish remover 1 | Prior art cream-like nail polish remover 2 | Prior art cream-like nail polish remover 3 |
|---|---|---|---|
| INCI name | propylene carbonate, peg-60 hydrogenated castor oil, hydrogenated rapeseed oil, ozokerite, copernicia cerifera (carnauba) wax, hydrogenated vegetable oil, stearyl stearate, stearic acid, prunus persica (peach) kernel oil, aleurites moluccana seed oil, tocopherol, dimethicone, tocopheryl acetate, peg-8, ascorbyl palmitate, ascorbic acid, citric acid, parfum (fragrance). | propylene carbonate, cera alba (beeswax /cire dabeille), peg-60 hydrogenated castor oil, hydrogenated rapeseed oil, copernicia cerifera cera (copernicia cerifera (carnauba) wax/ cire de carnauba), parfum (fragrance), dimethicone, tocopheryl acetate,cocos nucifera oil (cocos nucifera (coconut) oil), benzotriazolyl dodecyl p-cresol, peg-8, octyldodecyl stearoyl stearate, tocopherol, octyldodecanol, caprylyl glycol, ascorbyl palmitate, ascorbic acid, citric acid, phenoxyethanol, ci 77891 (titanium dioxide). | propylene carbonate, peg-40 hydrogenated castor oil, microcrystalline wax, hydrogenated castor oil, petrolatum, bisabolol, butyrospermum parkii butter, silica, parfum. |

Removing PEGs from nail polish remover compositions has been proven unsuccessful, since the resulting compositions are then not homogeneous and not stable.

The object of the present invention is to provide non-liquid nail polish remover, such as in the form of a cream or butter, that is environmental-friendly, not flammable, with a pleasant odour, efficient in removing nail polish and with moisturizing and repairing properties on nails, while being at the same time a homogeneous and stable composition.

### SUMMARY OF THE INVENTION

These and other objects are achieved by a nail polish remover composition and a method of removing polish from nails by using said composition, implemented according to the technical teachings of the appended claims.

### BRIEF DESCRIPTION OF THE FIGURES

The characteristics and advantages of the present invention will become apparent from the following detailed description, the embodiments provided by way of non-limiting examples and the figures annexed hereto, wherein:

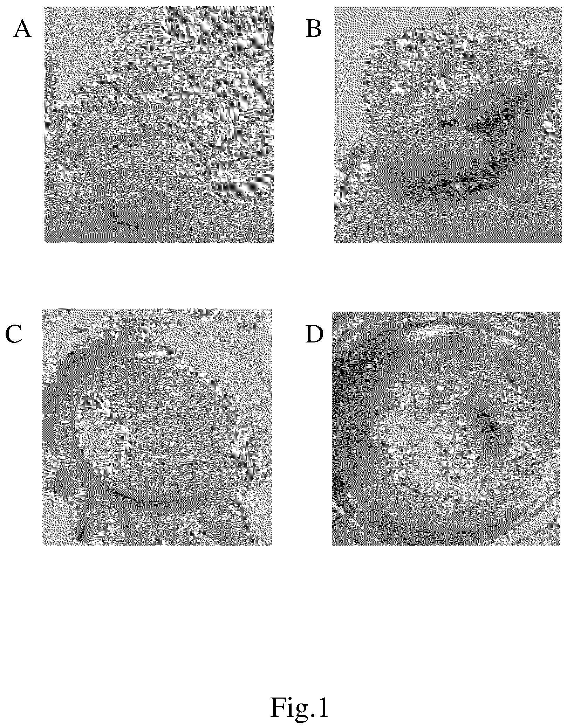

- Figure 1 shows the comparison between PEG-free cream-like nail polish remover compositions comprising succinate solvent according to the invention (Fig. 1 A and C) and carbonate solvent according to the prior art (Fig. 1 B, D), as per Example 2.

### DETAILED DESCRIPTION OF THE INVENTION

The nail polish remover composition of the present invention is in the form of a cream or butter, and comprises:
- a solvent, comprising at least a succinate ester;
- a viscosity controlling agent; and optionally
- cosmetically acceptable additives;
said nail polish remover composition optionally comprising one or more surfactants; wherein said nail polish remover composition does not include polyethylene glycols or derivatives thereof.

As said, with the term "polyethylene glycols (PEGs) or derivatives thereof' is meant PEGs and derivatives such as PEG-40 Hydrogenated Castor Oil and PEG-60 hydrogenated castor oil.

The instant nail polish remover in the form of a cream or butter has a number of advantages, such as:
- pleasant and natural odour
- non-flammable formula
- easily biodegradable
- up to 100% natural
- agreeable feeling during usage
- possibility to deliver it in tubes or jars
- low VOC and environmentally safe formula
- moisturizing and repairing effect on nails.

Moreover, it has been surprisingly found that the instant nail polish remover in the form of a cream or butter is homogenous and stable.

The term "homogeneous" means that the texture of the composition does not show lumps; the term "stable" means that the appearance of the composition is maintained over time. Actually, accelerated aging tests performed at 45°C in oven for a period of 3 months showed good stabilities. This was confirmed by the lack of liquid release from bulk, lack of phase separation, lack of lumps, and persistence of creamy consistency.

In preferred embodiments, the nail polish remover composition of the present invention is free of acetone and acetates.

In preferred embodiments, the nail polish remover composition of the present invention is free of alkylene carbonate, such as ethylene carbonate, propylene carbonate, butylene carbonate, trimethylene carbonate, and mixtures thereof.

The term "free of' referred to a component means that said component is not present in the composition, neither in traces.

With respect to the solvent, in the nail polish remover composition of the invention, said at least a succinate ester is preferably a dialkyl succinate. More preferably, said dialkyl succinate is diethyl succinate, dimethyl succinate, or a mixture thereof.

In preferred embodiments, said solvent is diethyl succinate. Diethyl succinate is a colorless liquid with gentle odour; it has good dissolving properties to nail varnishes, low VOC, high boiling point and it is easily biodegradable. Diethyl succinate has very good affinity to waxes, oils and non-polar compounds.

Preferably, said at least a succinate ester is of vegetable origin. For instance, the succinate ester can be diethyl succinate made by maize and beetroot.

Preferably, the nail polish remover composition comprises at least 40 wt%, more preferably 60-80 wt%, of solvent comprising at least a succinate ester, based on the weight of the composition.

In preferred embodiments, said at least a succinate ester is the sole solvent present in the nail polish remover composition of the invention.

Preferably, the nail polish remover composition is anhydrous, as no water is present therein.

Preferably, the nail polish remover composition comprises 1-60 wt%, more preferably 1-30 wt%, of a viscosity controlling agent, based on the weight of the composition. Said viscosity controlling agent of the composition is preferably selected from clay, silica, wax, a wax-like substance, hydrogenated oil and mixtures thereof. More preferably, said viscosity controlling agent comprises wax, hydrogenated oil or a mixture thereof. In most preferred embodiments, said viscosity controlling agent is wax, hydrogenated oil or a mixture thereof. Particularly preferred are embodiments wherein said viscosity controlling agent is a mixture of wax and hydrogenated oil.

Suitable cosmetically acceptable additives of the nail polish remover composition of the invention are preferably caring additives, such as emollients, vitamins, extracts, butters, and essential oils; cosmetic additives, such as component that strengthens or hardens nails, a component that moisturizes skin and nails, a component that hydrates cuticles, and a component that lowers loss of moisture from the skin, as well as skin conditioning agents, emollients, vitamins, extracts; aromas and perfumes.

Preferably, the nail polish remover composition of the invention comprises up to 40 wt% of cosmetic additives, based on the weight of the composition.

Preferably, the composition further comprises at least one component that strengthens or hardens nails, such as keratin amino acids, collagen, or ceramides.

Preferably, the composition further comprises at least one component that moisturizes skin and nails, such as oleyl lactate, linoleoyl lactate, linolenoyl lactate, decyl lactate, behenyl lactate, myristyl lactate, lauryl lactate, stearyl lactate, an ester of lactic acid and EPA alcohol (eicosapentaenyl alcohol), and an ester of lactic acid, DHA alcohol (docosahexaenyl alcohol), or a mixture thereof.

Preferably, the composition further comprises at least one component that hydrates cuticle, such as sodium hyaluronate, collagen, or a mixture thereof.

Preferably, the composition further comprises at least one component that lowers loss of moisture from the skin, such as cocoa butter, shea butter, other shea extracts, or a mixture thereof.

Furthermore, the nail polish remover composition of the invention preferably includes colouring agent(s), such as dye, pigments, pearlescent pigments.

Optionally, the nail polish remover composition of the present invention includes at least one surfactant, said surfactant(s) including nonionic, anionic, amphoteric, zwitterionic surfactants or mixtures thereof, but excluding polyethylene glycols or derivatives thereof, such as PEG-40 Hydrogenated Castor Oil and PEG-60 hydrogenated castor oil.

Preferably, the composition comprises up to 8 wt% of surfactant(s), based on the weight of the composition.

Suitable nonionic surfactants may be chosen, for example, from the group consisting of alkyl polyglucosides (APG), maltose esters, glucamine derivatives, for instance 2-ethylhexyl-oxycarbonyl-N-methylglucamine, and mixtures thereof. Alkylpolyglucosides that may be mentioned more particularly include decyl glucoside (alkyl-C9/C11-polyglucoside (1.4)), for instance the product sold under the name Mydol 10^{®} by the company Kao Chemicals, the product sold under the name Plantaren 2000 UP^{®} by the company Henkel, and the product sold under the name Oramix NS 10^{®} by the company SEPPIC; caprylyl/capryl glucoside, for instance the product sold under the name Oramix CG 110^{®} by the company SEPPIC; lauryl glucoside, for instance the products sold under the names Plantaren 1200 N^{®} and Plantacare 1200^{®} by the company Henkel; and cocoglucoside, for instance the product sold under the name Plantacare 818/UP^{®} by the company Henkel.

Suitable anionic surfactants may be chosen especially from carboxylates; amino acid derivatives such as sarcosinates and especially acylsarcosinates, for instance sodium lauroyl sarcosinate or sodium myristoyl sarcosinate; alkyl sulfates, such as MIPA-laureth sulfate(and)laureth-4, laureth-7, sodium laureth sulfate and their derivatives; alkyl ether sulfates, for instance sodium lauryl ether sulfate and ammonium lauryl ether sulfate; sulfonates such as, for example, α-olefin sulfonates; isethionates and acylisethionates, for instance sodium cocoylisethionate; taurates; sulfosuccinates; alkyl sulfoacetates; phosphates and alkyl phosphates, for instance lauryl phosphate; polypeptides; anionic derivatives of alkyl polyglucoside; fatty acid soaps, for instance the potassium or sodium salts of lauric, myristic, palmitic or stearic acid (potassium or sodium laurate, myristate, palmitate or stearate); and mixtures thereof.

Suitable amphoteric and zwitterionic surfactants may be chosen, for example, from betaines, for instance cocobetaine, laurylbetaine, oxyethylenated laurylbetaine or oxyethylenated stearylbetaine; N-alkylamidobetaines, for instance cocamidopropyl betaine; glycine derivatives, for instance sodium or potassium N-cocoylglycinate; sultaines, for instance cocoyl-amidopropylhydroxysulfobetaine; alkyl poly-aminocarboxylates; alkylamphoacetates, for instance N-disodium N-cocoyl-N-carboxymethoxyethyl-N-carboxy-methylethylenediamine and N-sodium N-cocoyl-N-hydroxy-ethyl-N-carboxymethylethylenediamine; and mixtures thereof.

When present in the nail polish remover composition of the invention, surfactant(s) are preferably selected from surfactants of natural source, for environmental safety reasons. In these embodiments, said surfactants are preferably selected from: coco glucoside, lauryl glucoside, decyl glucoside, caprylyl/capryl glucoside or a mixture thereof.

In accordance with the invention, when present, surfactants do not include polyethylene glycols or derivatives thereof.

In preferred embodiments, the nail polish remover composition of the invention comprises:
- at least 40 wt% of a solvent selected from diethyl succinate, dimethyl succinate and mixtures thereof,
- 1-60 wt% of a viscosity controlling agent, comprising wax, hydrogenated oil or a mixture thereof,
- 0-8 wt% of a surfactant,
- 0-5 wt% cosmetically acceptable additives,
based on the weight of the composition.

More preferably, the nail polish remover composition of the invention comprises:
- 60-80 wt% of a solvent selected from diethyl succinate, dimethyl succinate and mixtures thereof,
- 1-30 wt% of a viscosity controlling agent, comprising wax, hydrogenated oil or a mixture thereof,
- 0-8 wt% of a surfactant, and
- 0-5 wt% cosmetically acceptable additives,
based on the weight of the composition.

In some embodiments, the nail polish remover composition of the present invention - 60-80 wt% of a solvent selected from diethyl succinate, dimethyl succinate and mixtures thereof,
- 1-30 wt% of a viscosity controlling agent, comprising wax, hydrogenated oil or a mixture thereof,
- 0-8 wt% of a surfactant, and
- 0-5 wt% cosmetically acceptable additives,
based on the weight of the composition.

The expression "consists essentially of' means that the above listed components are the only components present in the composition of the invention, to be active in nail polish removing, whereas any other additive, excipient or ingredient, if present, does not interfere nor interact with said components.

Preferably, the nail polish remover composition of the invention only comprises components of natural origin.

Nail polish remover compositions, according to preferred embodiments of the present invention, are those in Tables 2 and 3 below.

**Table 2**

| Phase | INCI name | Function | Description | (wt %) |
|---|---|---|---|---|
| A | Diethyl Succinate | Solvent | Natural origin solvent derived from maize and beetroot | 66.85 |
| B | Polyglyceryl-6 Caprylate/Caprate (and) Sodium Caproyl/Lauroyl Lactylate | Surfactant solubilizer | Natural origin solubilizer, PEG-free | 8.00 |
| B | Copernicia Cerifera Cera | Film-forming/ Skin conditioning-emollient/ Viscosity controlling agent | Natural origin wax made from Copernicia Cerifera tree | 10.00 |
| B | Rhus Verniciflua Peel Cera | Skin conditioning-emollient/ Viscosity controlling agent | Natural origin wax derived from berry fruit peels of the Rhus Verniciflua tree | 6.00 |
| B | Hydrogenated Castor Oil Dimer Dilinoleate | Skin conditioning/ Viscosity controlling agent | Softening Texturizing agent | 4.00 |
| B | Hydrogenated Vegetable Oil | Viscosity controlling agent | Natural origin oil thickener | 4.00 |
| C | Coconut Alkanes | Skin conditioning-emollient | Natural origin silicone alternative | 1.00 |
| C | Helianthus Annuus Seed Oil, Tocopherol | Skin conditioning | Natural origin vit. E in oil | 0.15 |
| C | Parfum | Parfum | Natural origin perfume | 0-2 |
| C | Mica/ Beta Carotene / other | Colorant | Natural origin coloring agent | 0-1 |

**Table 3**

| Phase | INCI name | Function | Description | (wt %) |
|---|---|---|---|---|
| A | Diethyl Succinate | Solvent | Natural origin solvent derived from maize and beetroot | 72.75 |
| B | Copernicia Cerifera Cera | Film-forming/ Skin conditioning -emollient/ Viscosity controlling agent | Natural origin wax made from Copernicia Cerifera tree | 13.00 |
| B | Rhus Verniciflua Peel Cera | Skin conditioning-emollient/ Viscosity controlling agent | Natural origin wax derived from berry fruit peels of the Rhus Verniciflua tree | 7.00 |
| B | Hydrogenated Vegetable Oil | Viscosity controlling agent | Natural origin oil thickener | 5.00 |
| C | Crambe Abyssinica Seed Oil, Euphorbia Cerifera Cera, Hydroxystearic Acid, Beta-Sitosterol, Rhus Verniciflua Peel Cera/Rhus Succedanea Fruit Cera, Tocopherol, Helianthus Annuus Seed Oil | Skin conditioning | Softening Texturizing agent | 0.50 |
| C | Helianthus Annuus Seed Oil, Tocopherol | Skin conditioning | Natural origin vit. E in oil | 0.15 |
| C | Parfum | Parfum | Natural origin perfume | 0-2 |
| C | Mica/ Beta Carotene / other | Colorant | Natural origin coloring agent | 0-1 |

The nail polish remover compositions of Tables 2 and 3 are creamy compositions, comprising only components of natural origin.

The present invention also relates to a process for preparing the above described nail polish remover composition. Said process comprises the steps of:
i) heating and mixing the viscosity controlling agent up to 85°C,
ii) separately heating the solvent up to 80 C,
iii) adding the heated solvent of step ii) to the heated viscosity controlling agent of step i) under stirring, to obtain a mixture,
iv) cooling down to 60°C the mixture of step iii), and optionally adding the cosmetically acceptable additives,
v) homogenizing for 5 minutes by using high speed homogenizer.

In preferred embodiment, the viscosity controlling agent of step i) is a mix of the viscosity controlling agents of phase B in Table 2 or 3 above.

In preferred embodiment, the solvent of step ii) is the solvent of phase A in Table 2 or 3 above.

In preferred embodiment, the cosmetically acceptable additives of step iv) is a mix of the cosmetically acceptable additives of phase C in Table 2 or 3 above.

In the most preferred embodiments of the process:
the viscosity controlling agent of step i) is a mix of the viscosity controlling agents of phase B in Table 2 above,
the solvent of step ii) is the solvent of phase A in Table 2 above, and
the cosmetically acceptable additives of step iv) is a mix of the cosmetically acceptable additives of phase C in Table 2 above;
   or
the viscosity controlling agent of step i) is a mix of the viscosity controlling agents of phase B in Table 3 above,
the solvent of step ii) is the solvent of phase A in Table 3 above, and
the cosmetically acceptable additives of step iv) is a mix of the cosmetically acceptable additives of phase C in Table 3 above.

The present invention also relates to a method of removing polish from nails by using the nail polish remover composition, as above described.

The method comprises the steps of:
a) applying the composition in the form of a cream or butter onto a varnished nail,
b) letting the composition act for 15-30 seconds, and
c) removing the composition and the polish by using a pad soaked with the composition.

The composition of the invention allows to remove regular nail polishes whether containing pigments or not, where nitrocellulose and its derivative are present as a film former. However, in addition, the composition of the invention advantageously allows to remove also water-based nail polishes developed with polymers, such us polymethyl methacrylate and polyurethane-2, acrylic copolymers as film formers.

The nail polish remover composition is in the form of a cream or butter at room temperature, so that it can be directly applicable on nails, for example by using a dispenser.

The nail polish remover composition can be contained in a flexible container, such as a tube, and dispensed squeezing the container, or it can be contained in a rigid container and dispensed by using a spatula.

It should be understood that all aspects identified as preferred and advantageous for the composition are to be deemed as similarly preferred and advantageous also for the uses, the preparation and method of application of the same.

It should be also understood that all the combinations of preferred aspects of the composition of the invention, as well as of uses, the preparation and method of application, as above reported, are to be deemed as hereby disclosed.

Below are working examples of the present invention provided for illustrative and non-limiting purposes.

### EXAMPLES

### Example 1

Comparative nail polish remover compositions in the form of cream have been prepared using propylene carbonate as solvent and with several different non-PEG surfactants of natural origin.

Table 4 below shows the surfactants used in each comparative composition, the concentration of surfactants (% by weight based on the weight of the composition) and the appearance of the compositions obtained (Result).

**Table 4**

| Comparative Compositions | Surfactants | | | Result |
|---|---|---|---|---|
| | Trade Name | INCI name | wt% | |
| 1 | Symsol PF-3 | Water (Aqua), Pentylene Glycol, Sodium Lauryl Sulfoacetate, Sodium Oleoyl Sarcosinate, Sodium Chloride, Sodium Oleate | 11 | Not homogeneous |
| 2 | Sepiclear G7 | Heptyl Glucoside | 11 | Not homogeneous |
| 3 | Tego Care Ltp | Sorbitan Laurate (and) Polyglyceryl-4 Laurate (and) Dilauryl Citrate | 11 | Not homogeneous |
| 4 | Viscaress Hpd | Polyquaternium-37 (and) Hydrogenated Polydecene (and) Trideceth-6 | 11 | Not homogeneous |
| 5 | TWEEN 80LQ | Polysorbate 80 | 11, 19, 23 | Lumps in the formula, not stable in time |
| 6 | Tween 20LQ | Polysorbate 20 | 15, 19 | Lumps in the formula, not stable in time |
| 7 | Durosoft Pg10cy | Polyglyceryl-10 Caprylate | 11 | Not homogeneous |
| 8 | Resassol Apostrophie | Caprylyl/Capryl Glucoside (and) Water (and) Polyglyceryl-3 Cocoate (and) Polyglyceryl-10 Laurate (and) Citric Acid | 19 | Not homogeneous |
| 9 | Transcutol CG | Ethoxydiglycol | 19 | Not homogeneous |
| 10 | IMWITOR Litemuls | Glyceryl Citrate/Lactate/Linoleate/ Oleate, Polyglyceryl-4 Cocoate, Polyglyceryl-3 Caprate, Glyceryl Caprylate | 23 | Not homogeneous |
| 11 | Easy Tens S2 | Polyglyceryl-6 Caprylate/Caprate (and) Sodium Caproyl/Lauroyl Lactylate | 11 | Not homogeneous |
| 12 | Easy Tens S2 Pure | Polyglyceryl-6 Caprylate | 11 | Not homogeneous |

None of the compositions obtained with propylene carbonate as solvent and non-PEG surfactants is satisfactory in terms of homogeneity and stability.

### Example 2

Nail polish remover PEG-free compositions in the form of cream have been prepared including either diethyl succinate (Inventive Composition of Table 5) as solvent or propylene carbonate (Comparative Composition of Table 6).

**Table 5**

| INCI name | Function | Description | (wt %) |
|---|---|---|---|
| Diethyl Succinate | Solvent | Natural origin solvent derived from maize and beetroot | 75.00 |
| Copernicia Cerifera Cera | Film-forming/ Skin conditioning -emollient Viscosity controlling | Natural origin wax made from Copernicia Cerifera tree | 13.00 |
| Rhus Verniciflua Peel Cera | Skin conditioning-emollient Viscosity controlling | Natural origin wax derived from berry fruit peels of the Rhus Verniciflua tree | 7.00 |
| Hydrogenated Vegetable Oil | Viscosity controlling | Natural origin oil thickener | 5.00 |

**Table 6**

| INCI name | Function | Description | (wt %) |
|---|---|---|---|
| Propylene Carbonate | Solvent | Synthetic solvent | 75.00 |
| Copernicia Cerifera Cera | Film-forming/ Skin conditioning -emollient Viscosity controlling | Natural origin wax made from Copernicia Cerifera tree | 13.00 |
| Rhus Verniciflua Peel Cera | Skin conditioning-emollient Viscosity controlling | Natural origin wax derived from berry fruit peels of the Rhus Verniciflua tree | 7.00 |
| Hydrogenated Vegetable Oil | Viscosity controlling | Natural origin oil thickener | 5.00 |

The two compositions have been compared for their appearance, consistency and stability in time.

Appearance of the compositions can be seen on Fig. 1, wherein Fig. 1A, and 1C show the composition according to the invention and Fig. 1B, and ID show the comparative composition.

Consistency and stability are reported in Table 7 below.

**Table 7**

| Description | Composition of Table 5, according to the invention | Composition of Table 6, comparative |
|---|---|---|
| Consistency | Homogeneous | Non- homogeneous |
| Stability in time | Stable | Non-stable |

### Example 3

The following nail polish remover composition in cream has been prepared:

**Table 8**

| INCI name | Function | Description | (wt %) |
|---|---|---|---|
| Diethyl Succinate | Solvent | Natural origin solvent derived from maize and beetroot | 66.85 |
| Polyglyceryl-6 Caprylate/Caprate (and) Sodium Caproyl/Lauroyl Lactylate | Surfactant solubilizer | Natural origin solubilizer, PEG-free | 8.00 |
| Copernicia Cerifera Cera | Film-forming/ Skin conditioning -emollient Viscosity controlling | Natural origin wax made from Copernicia Cerifera tree | 10.00 |
| Rhus Verniciflua Peel Cera | Skin conditioning-emollient Viscosity controlling | Natural origin wax derived from berry fruit peels of the Rhus Verniciflua tree | 6.00 |
| Hydrogenated Castor Oil Dimer Dilinoleate | Skin conditioning Viscosity controlling | Softening Texturizing agent | 4.00 |
| Hydrogenated Vegetable Oil | Viscosity controlling | Natural origin oil thickener | 4.00 |
| Coconut Alkanes | Skin conditioning-emollient | Natural origin silicone alternative | 1.00 |
| Helianthus Annuus Seed Oil, Tocopherol | Skin conditioning | Natural origin vit. E in oil | 0.15 |
| Parfum | Parfum | Natural origin perfume | 0-2 |
| Mica/ CI | Colorant | Natural origin coloring agent | 0-1 |

All the components have been mixed by using a standard agitator.

### Example 4

The following nail polish remover composition in cream has been prepared:

**Table 9**

| INCI name | Function | Description | (wt %) |
|---|---|---|---|
| Diethyl Succinate | Solvent | Natural origin solvent derived from maize and beetroot | 72.75 |
| Copernicia Cerifera Cera | Film-forming/ Skin conditioning -emollient Viscosity controlling | Natural origin wax made from Copernicia Cerifera tree | 13.00 |
| Rhus Verniciflua Peel Cera | Skin conditioning-emollient Viscosity controlling | Natural origin wax derived from berry fruit peels of the Rhus Verniciflua tree | 7.00 |
| Hydrogenated Vegetable Oil | Viscosity controlling | Natural origin oil thickener | 5.00 |
| Crambe Abyssinica Seed Oil, Euphorbia Cerifera Cera, Hydroxystearic Acid, Beta-Sitosterol, Rhus Verniciflua Peel Cera/Rhus Succedanea Fruit Cera, Tocopherol, Helianthus Annuus Seed Oil | Skin conditioning | Softening Texturizing agent | 0.50 |
| Helianthus Annuus Seed Oil, Tocopherol | Skin conditioning | Natural origin vit. E in oil | 0.15 |
| Parfum | Parfum | Natural origin perfume | 0-2 |
| Mica/ CI | Colorant | Natural origin coloring agent | 0-1 |

All the components have been mixed by using a standard agitator.

## Claims

1. A nail polish remover composition in the form of a cream or butter, and comprising:
- a solvent, comprising at least a succinate ester;
- a viscosity controlling agent; and optionally
- cosmetically acceptable additives;
said nail polish remover composition optionally comprising one or more surfactants;
wherein said nail polish remover composition does not include polyethylene glycols or derivatives thereof.

2. The nail polish remover composition of claim 1, comprising at least 40 wt%, preferably 60-80 wt%, of a solvent, based on the weight of the composition.

3. The nail polish remover composition of claims 1 or 2, wherein said at least a succinate ester is diethyl succinate, dimethyl succinate, or a mixture thereof.

4. The nail polish remover composition of any one of claims 1-3, being free of at least one of acetone, acetates, alkylene carbonate or of mixtures thereof.

5. The nail polish remover composition of any one of claims 1-4, comprising 1-60 wt%, more preferably 1-30 wt%, of a viscosity controlling agent, based on the weight of the composition.

6. The nail polish remover composition of any one of claims 1-5, wherein said viscosity controlling agent comprises wax, hydrogenated oil or a mixture thereof.

7. The nail polish remover composition of any one of claims 1-6, comprising:
- at least 40 wt% of a solvent selected from diethyl succinate, dimethyl succinate and mixtures thereof,
- 1-60 wt% of a viscosity controlling agent, comprising wax, hydrogenated oil or a mixture thereof,
- 0-8 wt% of a surfactant,
- 0-5 wt% cosmetically acceptable additives,
based on the weight of the composition.

8. The nail polish remover composition of claims 1-7, comprising:
- 60-80 wt% of a solvent selected from diethyl succinate, dimethyl succinate and mixtures thereof,
- 1-30 wt% of a viscosity controlling agent, comprising wax, hydrogenated oil or a mixture thereof,
- 0-8 wt% of a surfactant, and
- 0-5 wt% cosmetically acceptable additives,
based on the weight of the composition.

9. A process for preparing the nail polish remover composition of claims 1-8, said process comprising the steps of:
i) heating and mixing the viscosity controlling agent up to 85°C,
ii) separately heating the solvent up to 80 C,
iii) adding the heated solvent of step ii) to the heated viscosity controlling agent of step i) under stirring, to obtain a mixture,
iv) cooling down to 60°C the mixture of step iii), and optionally adding the cosmetically acceptable additives,
v) homogenizing for 5 minutes by using high speed homogenizer.

10. A method of removing polish from nails by using the nail polish remover composition of claim 1, the method comprising the steps of:
a) applying the composition of claims 1-8 onto a varnished nail,
b) letting the composition act for 15-30 seconds, and
c) removing the composition and the polish by using a pad soaked with the composition.
